# EUROPEAN PATENT APPLICATION

(11) **EP 4 582 116 A1**
(43) Date of publication of application: **09.07.2025**
(21) Application number: 25150173.0
(22) Date of filing: 03.01.2025
(51) Int. Cl.: A61M 1/36

(54) **CONTROL METHOD, DEVICE, EQUIPMENT AND MEDIUM FOR ABNORMAL ALARM IN BLOOD BUBBLE MONITORING**

(30) Priority: 05.01.2024 CN 202410018965
(71) Applicant: ChinaBridge (Shenzen) Medical Technology Co., Ltd., Shenzhen, Guangdong 518102 (CN)
(72) Inventor: LI, Yijiang, Shenzhen Guangdong, 518102 (CN)
(74) Representative: Pfenning, Meinig & Partner mbB

(57) **Abstract**

A method for abnormal alarm in blood bubble monitoring includes: when receiving a bubble signal sent by a bubble sensor, obtaining the first moment when the bubble signal is generated, and obtaining pressure sampling data of the extracorporeal circulation pipeline within a preset target time range based on a pressure acquisition device. Based on the pressure sampling data, judging whether there is abnormal pressure fluctuation in the extracorporeal circulation pipeline within the target time range. If so, obtaining the abnormal fluctuation time range corresponding to the abnormal pressure fluctuation in the extracorporeal circulation pipeline. Judging whether the first moment is within the abnormal fluctuation time range. If so, ignoring the bubble signa. If the first moment is not within the abnormal fluctuation time range, or if there is no abnormal pressure fluctuation in the extracorporeal circulation pipeline within the target time range, issuing a bubble alarm message.

## Description

### TECHNICAL FIELD

The present invention relates to the field of medical monitoring technology, and in particular to a control method, device, equipment and medium for abnormal alarm in blood bubble monitoring.

### BACKGROUND

In extracorporeal blood bubble monitoring, for example, Extracorporeal Membrane Oxygenation (ECMO) systems are equipped with bubble monitoring sensors. A conventional bubble sensor uses ultrasonic signals to monitor bubbles in the hose line. Since the sensor is clamped on the outer wall of the hose line during use, in actual clinical processes, if the hose line shakes (for example, during transportation or nursing), a gap will be created between the ultrasonic probe and the hose line, resulting in false alarms in bubble monitoring.

Usually, the ECMO system will generate an alarm when it detects bubbles. When a bubble alarm is found in clinical practice, the measures taken are to clamp the pipeline and stop the pump to expel the bubbles. If the bubble sensor falsely alarms, it will cause medical staff to perform unnecessary operations, resulting in reduced work efficiency.

In the prior art, one method is to add a coupling agent between the ultrasonic probe and the pipeline to prevent the air in the gap from reflecting the ultrasonic signal. Adding a coupling agent is a traditional ultrasonic monitoring method. The coupling agent is easy to evaporate and dry out after long-term use, and it brings many inconveniences to cleaning and disinfection. It has been gradually eliminated from clinical use.

### SUMMARY

The embodiments of the present invention provide a control method, device, equipment and medium for abnormal alarm in blood bubble monitoring, aiming to solve the problem of inaccurate blood bubble monitoring in an extracorporeal circulation hose circuit and easy false alarm in the prior art.

In a first aspect, an embodiment of the present invention provides a control method for abnormal alarm in blood bubble monitoring, wherein an extracorporeal circulation pipeline of an extracorporeal circulation device is provided with a bubble sensor and a pressure acquisition device, and the method comprises:

When receiving the bubble signal sent by the bubble sensor, obtaining the first moment when the bubble signal is generated, and obtaining the pressure sampling data of the extracorporeal circulation pipeline within a preset target time range based on the pressure acquisition device, wherein the first moment is within the target time range;

Based on the pressure sampling data, determining whether there is abnormal pressure fluctuation in the extracorporeal circulation circuit within the target time range;

If there is abnormal pressure fluctuation in the extracorporeal circulation pipeline within the target time range, obtaining an abnormal fluctuation time range corresponding to the abnormal pressure fluctuation in the extracorporeal circulation pipeline;

Determining whether the first moment is within the abnormal fluctuation time range;

If the first moment is within the abnormal fluctuation time range, ignoring the bubble signal;

If the first moment is not within the abnormal fluctuation time range, or if there is no abnormal pressure fluctuation in the extracorporeal circulation circuit within the target time range, a bubble alarm message is issued.

A further technical solution is that the pressure sampling data includes pressure values at multiple different sampling times, and judging whether there is abnormal pressure fluctuation in the extracorporeal circulation circuit within the target time range based on the pressure sampling data includes:

Performing linear fitting based on the sampling time and pressure value in the pressure sampling data to obtain a fitting result;

Determining, according to the fitting result, whether the pressure value in the pressure sampling data changes linearly with the sampling time;

If the pressure value in the pressure sampling data changes linearly with the sampling time, it is determined that there is no abnormal pressure fluctuation in the extracorporeal circulation circuit within the target time range.

A further technical solution is that judging whether there is abnormal pressure fluctuation in the extracorporeal circulation circuit within the target time range based on the pressure sampling data further includes:

If the pressure value in the pressure sampling data presents a nonlinear change with the sampling time, obtaining a maximum deviation value of the pressure fluctuation in the pressure sampling data;

Determining whether the maximum deviation value of the pressure fluctuation is greater than a preset deviation threshold;

If the maximum deviation value of the pressure fluctuation is greater than a preset deviation threshold, it is determined that there is no abnormal pressure fluctuation in the extracorporeal circulation circuit within the target time range;

If the maximum deviation value of the pressure fluctuation is not greater than a preset deviation threshold, it is determined that abnormal pressure fluctuation exists in the extracorporeal circulation circuit within the target time range.

A further technical solution is that the fitting result includes a correlation coefficient, and judging whether the pressure value in the pressure sampling data presents a linear change with the sampling time according to the fitting result includes:

Determine whether the difference between the absolute value of the correlation coefficient and 1 is less than a preset difference threshold;

If the difference between the absolute value of the correlation coefficient and 1 is less than a preset difference threshold, it is determined that the pressure value in the pressure sampling data changes linearly with the sampling time;

If the difference between the absolute value of the correlation coefficient and 1 is not less than a preset difference threshold, it is determined that the pressure value in the pressure sampling data presents a nonlinear change with the sampling time.

A further technical solution is that the fitting result also includes a linear regression equation, and the abnormal fluctuation time range corresponding to the abnormal pressure fluctuation in the extracorporeal circulation circuit is obtained, including:

Calculate the regression pressure value corresponding to the sampling time based on the linear regression equation;

If the difference between the pressure value corresponding to the sampling time and the regression pressure value is greater than a preset pressure value threshold, it is determined that the pressure value corresponding to the sampling time is an abnormal pressure value;

The sampling time corresponding to the earliest abnormal pressure value within the target time range is taken as the starting point of the abnormal fluctuation time range, and the sampling time corresponding to the latest abnormal pressure value within the target time range is taken as the end point of the abnormal fluctuation time range.

A further technical solution is that the step of obtaining the maximum deviation value of the pressure fluctuation in the pressure sampling data includes:

The maximum value of the difference between all abnormal pressure values and the corresponding regression pressure values is obtained as the maximum deviation value of the pressure fluctuation in the pressure sampling data.

A further technical solution is that the pressure acquisition device includes a plurality of pressure sensors, and the pressure sampling data of the extracorporeal circulation circuit within a preset target time range is obtained based on the pressure acquisition device, including:

From the plurality of pressure sensors, obtaining a pressure sensor that is closest to the bubble sensor as a target pressure sensor;

The pressure data of the target pressure sensor within a preset target time range is acquired as the pressure sampling data.

A further technical solution is that a flow sensor is further provided on the extracorporeal circulation pipeline of the extracorporeal circulation device, and before ignoring the bubble signal, the method further comprises:

Acquire flow sampling data of the flow sensor within the abnormal fluctuation time range;

Based on the flow sampling data, determining whether there is flow fluctuation in the extracorporeal circulation pipeline within the abnormal fluctuation time range;

If there is flow fluctuation in the extracorporeal circulation pipeline within the abnormal fluctuation time range, a bubble alarm message is issued;

If there is no flow fluctuation in the extracorporeal circulation circuit within the abnormal fluctuation time range, the step of ignoring the bubble signal is performed.

A further technical solution is that the pressure sampling period within the target time range is 100-1000 milliseconds, and the length of the target time range is 5-90 seconds.

In a second aspect, an embodiment of the present invention further provides a control device for abnormal alarm in blood bubble monitoring, which includes a unit for executing the above method.

In a third aspect, an embodiment of the present invention further provides a computer device, which includes a memory and a processor, wherein a computer program is stored in the memory, and the processor implements the above method when executing the computer program.

In a fourth aspect, an embodiment of the present invention further provides a computer-readable storage medium, wherein the storage medium stores a computer program, and the computer program can implement the above method when executed by a processor.

The embodiment of the present invention provides a control method, device, equipment and medium for abnormal alarm in blood bubble monitoring. The method includes: when receiving the bubble signal sent by the bubble sensor, obtaining the first moment when the bubble signal is generated, obtaining the pressure sampling data of the extracorporeal circulation pipeline within a preset target time range based on the pressure acquisition device, wherein the first moment is within the target time range; based on the pressure sampling data, judging whether there is abnormal pressure fluctuation in the extracorporeal circulation pipeline within the target time range; if there is abnormal pressure fluctuation in the extracorporeal circulation pipeline within the target time range, obtaining the abnormal fluctuation time range corresponding to the abnormal pressure fluctuation in the extracorporeal circulation pipeline; judging whether the first moment is within the abnormal fluctuation time range; if the first moment is within the abnormal fluctuation time range, ignoring the bubble signal; if the first moment is not within the abnormal fluctuation time range, or if there is no abnormal pressure fluctuation in the extracorporeal circulation pipeline within the target time range, issuing a bubble alarm message. The technical solution of the present invention, when receiving the bubble signal sent by the bubble sensor, determines based on the pressure information of the extracorporeal circulation pipeline whether there is abnormal pressure fluctuation, that is, pressure fluctuation caused by shaking, in the extracorporeal circulation pipeline at the first moment when the bubble signal is detected. If so, the bubble signal is ignored and no bubble alarm information is issued, thereby effectively avoiding false alarms of bubble warnings and greatly improving the accuracy of bubble detection.

### BRIEF DESCRIPTION OF THE DRAWINGS

In order to more clearly illustrate the technical solutions of the embodiments of the present invention, the accompanying drawings required for use in the description of the embodiments will be briefly introduced below. Obviously, the accompanying drawings described below are some embodiments of the present invention. For ordinary technicians in this field, other embodiments can be obtained based on these accompanying drawings without undue experimentation.
FIG. 1 is a flow chart of a control method for abnormal alarm in blood bubble monitoring provided by an embodiment of the present invention;
FIG. 2 is a schematic diagram of an application scenario of an extracorporeal circulation circuit provided by an embodiment of the present invention;
FIG. 3 is a schematic block diagram of a control device for abnormal alarm in blood bubble monitoring provided by an embodiment of the present invention;
FIG. 4 is a schematic block diagram of a computer device provided in an embodiment of the present invention.

### DETAILED DESCRIPTION

The following will be combined with the drawings in the embodiments of the present invention to clearly and completely describe the technical solutions in the embodiments of the present invention. The described embodiments are some of the embodiments of the present invention, not all of the embodiments. Based on the embodiments of the present invention, all other embodiments obtained by ordinary technicians in this field without creative work are within the scope of protection of the present invention.

It should be understood that when used in this specification and the appended claims, the terms "include" and "comprises" indicate the presence of described features, integers, steps, operations, elements and/or components, but do not exclude the presence or addition of one or more other features, integers, steps, operations, elements, components and/or combinations thereof.

It should also be understood that the terms used in this specification of the present invention are only for the purpose of describing specific embodiments and are not intended to limit the present invention. As used in the specification of the present invention and the appended claims, unless the context clearly indicates otherwise, the singular forms "a", "an" and "the" are intended to include plural forms.

It should be further understood that the term "and/or" used in the present description and the appended claims refers to and includes any and all possible combinations of one or more of the associated listed items.

As used in this specification and the appended claims, the term "if" may be interpreted as "when" or "upon" or "in response to determining" or "in response to detecting," depending on the context. Similarly, the phrases "if it is determined" or "if [described condition or event] is detected" may be interpreted as meaning "upon determination" or "in response to determining" or "upon detection of [described condition or event]" or "in response to detecting [described condition or event]," depending on the context.

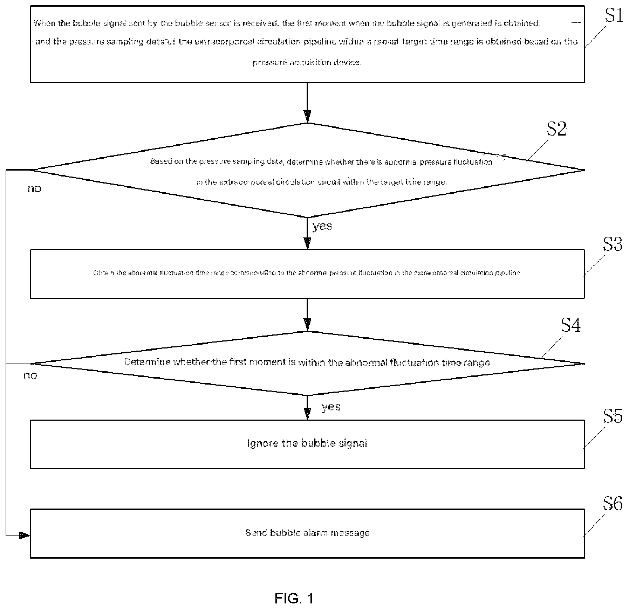

Referring to FIG. 1, an embodiment of the present invention provides a control method for abnormal alarm in blood bubble monitoring. The method is applied to an extracorporeal circulation device. An extracorporeal circulation pipeline of the extracorporeal circulation device (such as an ECMO device) is provided with a bubble sensor and a pressure acquisition device. The bubble sensor is used to detect bubbles in the extracorporeal circulation pipeline, and the pressure acquisition device is used to collect the pressure of the extracorporeal circulation pipeline. As shown in FIG. 1, the method includes the following steps:

Step S1: when receiving the bubble signal sent by the bubble sensor, obtain the first moment when the bubble signal is generated, and obtain the pressure sampling data of the extracorporeal circulation circuit within a preset target time range based on the pressure acquisition device, wherein the first moment is within the target time range.

In specific implementation, usually, the bubble sensor will send a bubble signal when it detects bubbles. However, there may be some misjudgments. For example, when the extracorporeal circulation pipeline shakes, a gap will appear between the bubble sensor and the pipeline, resulting in misjudgment. Embodiments of the present invention eliminate the above misjudgment and improve the accuracy of bubble detection.

Specifically, when the bubble signal sent by the bubble sensor is received, the time corresponding to the receipt of the bubble signal is taken as the first time.

The pressure acquisition device continuously acquires the pressure of the extracorporeal circulation circuit. For example, the pressure value of the extracorporeal circulation circuit is acquired once at every preset pressure sampling period, and the pressure sampling data acquired within a period of time (for example, 2 minutes, which is not specifically limited in the present invention) is continuously stored.

Then, the target time range is determined based on the first moment. The principle is that the first moment must be within the target time range. In some embodiments, the first moment may be at the end of the target time range. In order to meet the real-time nature of the bubble alarm, it is necessary to review as many pressure change trends as possible in the previous period to determine the pressure fluctuation. Therefore, the first moment needs to be at the end of the target time range.

Further, the length of the target time range is 5-90 seconds, for example, 60 seconds. Further, the pressure sampling period within the target time range is 100-1000 milliseconds. The pressure sampling data within the target time range is extracted from the collected pressure sampling data. If the sampling period is too short, the number of operations will be large, and the pressure values collected will fluctuate greatly; if the sampling period is too long, such as exceeding 1 second, the instantaneous pressure fluctuations may not be monitored, and missed judgments may occur. In some embodiments of the present invention, the sampling period is approximately 200 milliseconds.

If the target time range is too short, the linearity of the fitted data will be poor. If the target time range is too long, it will lead to a waste of computing resources and computing time. In some embodiments of the present invention, the target time range is approximately 60 seconds.

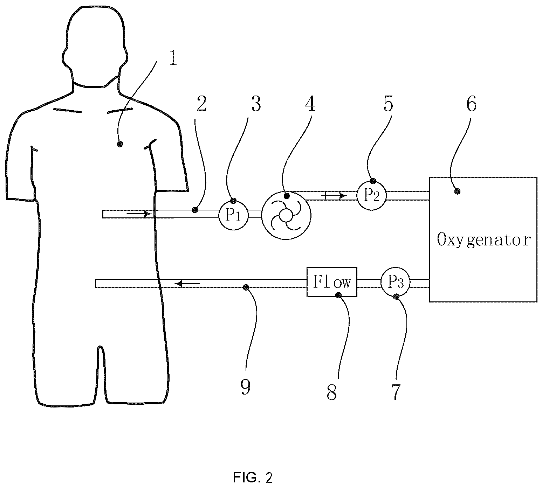

Referring to FIG. 2, in one embodiment, the pressure acquisition device includes a plurality of pressure sensors. For example, in the extracorporeal circulation circuit shown in FIG. 2, the pressure acquisition device includes a first pressure sensor 3 and a second pressure sensor 5 respectively located at both ends of a centrifugal pump 4 on a drainage circuit 2, and a third pressure sensor 7 located on a perfusion circuit 9 at the outlet end of an oxygenator 6. Meanwhile, a bubble sensor 8 is provided on the downstream side of the third pressure sensor 7. Both the drainage circuit 2 and the perfusion circuit 9 are connected to the patient 1.

In some embodiments, the above step S1 (shown in FIG. 1) of "obtaining pressure sampling data of the extracorporeal circulation circuit within a preset target time range based on the pressure acquisition device" specifically includes the following steps:

Step S11 (not shown): setting, from the plurality of pressure sensors, a pressure sensor that is closest to the bubble sensor as a target pressure sensor.

In a specific implementation, the pressure sensor closest to the bubble sensor is obtained from the multiple pressure sensors as the target pressure sensor. For example, in this embodiment, the third pressure sensor is closest to the bubble sensor and is located in the same perfusion pipeline, so the third pressure sensor is selected as the target pressure sensor.

Step S12 (not shown): obtaining pressure data of the target pressure sensor within a preset target time range as the pressure sampling data.

In a specific implementation, the pressure detected by the target pressure sensor is closest to the pressure at the bubble sensor. Therefore, in the present invention, the pressure data of the target pressure sensor within a preset target time range is used as the pressure sampling data.

Referring back to FIG. 1, step S2: based on the pressure sampling data, determine whether there is abnormal pressure fluctuation in the extracorporeal circulation circuit within the target time range.

In a specific implementation, when the extracorporeal circulation pipeline is not shaking abnormally, the pressure inside it changes linearly. By fitting the measured pressure data of the extracorporeal circulation pipeline, when the pipeline is normal and without shaking, the pressure data shows a linear change trend. The equation Y = AX + B can be fitted.

Where Y is the pressure value, X is the time, and A and B are the constants of the fitting formula.

When the pressure does not fluctuate, during the fitting process, if the pressure in the extracorporeal circulation circuit is relatively stable, the A value is close to zero, and the B value is close to the pressure value of the extracorporeal circulation circuit. If the pressure value in the extracorporeal circulation circuit is in a gradual upward trend, the A value is a positive value; if the pressure value in the extracorporeal circulation circuit is in a gradual downward trend, the A value is a negative value; but its overall trend still has a linear relationship. Therefore, when fitting under different pressure value changes, the A value and the B value are not fixed, but can change through the pressure value fitting, and overall, there is still a linear regularity. When the pipeline shakes and causes the pressure value to fluctuate, during the fitting process, there will be obvious pressure values that do not conform to the linear relationship. Therefore, the change of pressure in the extracorporeal circulation circuit over time can be used to determine whether there is abnormal pressure fluctuation (abnormal shaking, etc.) in the extracorporeal circulation circuit.

In one embodiment, the pressure sampling data includes pressure values at multiple different sampling times, and the above step S2 of "determining whether there is abnormal pressure fluctuation in the extracorporeal circulation circuit within the target time range based on the pressure sampling data" specifically includes the following steps:

Step S21 (not shown): performing linear fitting based on the sampling time and pressure value in the pressure sampling data to obtain a fitting result.

In a specific implementation, the sampling time is used as an independent variable and the pressure value is used as a dependent variable, and a linear fitting is performed on the sampling time and the pressure value in the pressure sampling data to obtain a fitting result.

The fitting results include a correlation coefficient and a linear regression equation. The correlation coefficient is used to characterize the linear correlation between the sampling time and the pressure value in the pressure sampling data. The linear regression equation is used to characterize the variation law between the sampling time and the pressure value in the pressure sampling data.

Step S22 (not shown): judging, according to the fitting result, whether the pressure value in the pressure sampling data changes linearly with the sampling time.

In a specific implementation, it is usually determined whether the pressure value in the pressure sampling data presents a linear change with the sampling time according to the correlation coefficient in the fitting result. The closer the absolute value of the correlation coefficient is to 1, the higher the degree of linear correlation is. The closer the absolute value of the correlation coefficient is to 0, the lower the degree of linear correlation is.

For example, in one embodiment, the above step S22 of "determining whether the pressure value in the pressure sampling data presents a linear change with the sampling time according to the fitting result" specifically includes:

Step S221 (not shown), determining whether the difference between the absolute value of the correlation coefficient and 1 is less than a preset difference threshold.

In a specific implementation, the difference between the absolute value of the correlation coefficient and 1 is calculated (the difference here refers to the absolute value of the difference between the absolute value of the correlation coefficient and 1), and it is determined whether the difference is less than a preset difference threshold. The difference threshold can be set by a person skilled in the art, and the present invention does not specifically limit it, for example, it is set to 0.05.

Step S222 (not shown): If the difference between the absolute value of the correlation coefficient and 1 is less than a preset difference threshold, it is determined that the pressure value in the pressure sampling data changes linearly with the sampling time.

In a specific implementation, if the difference between the absolute value of the correlation coefficient and 1 is smaller than a preset difference threshold, it is determined that the pressure value in the pressure sampling data presents a linear change with the sampling time.

Step S223 (not shown): If the difference between the absolute value of the correlation coefficient and 1 is not less than a preset difference threshold, it is determined that the pressure value in the pressure sampling data presents a nonlinear change with the sampling time.

In a specific implementation, if the difference between the absolute value of the correlation coefficient and 1 is not less than a preset difference threshold, it is determined that the pressure value in the pressure sampling data presents a nonlinear change with the sampling time.

Step S23 (not shown): If the pressure value in the pressure sampling data changes linearly with the sampling time, it is determined that there is no abnormal pressure fluctuation in the extracorporeal circulation circuit within the target time range.

In a specific implementation, if the pressure value in the pressure sampling data changes linearly with the sampling time, it means that there is no abnormal shaking in the extracorporeal circulation pipeline within the target time range. Therefore, it is determined that there is no abnormal pressure fluctuation in the extracorporeal circulation pipeline within the target time range.

Step S24 (not shown): If the pressure value in the pressure sampling data presents a nonlinear change with the sampling time, obtain a maximum deviation value of the pressure fluctuation in the pressure sampling data.

In a specific implementation, if the pressure value in the pressure sampling data shows a nonlinear change with the sampling time, it means that there is pressure fluctuation in the extracorporeal circulation pipeline within the target time range, and there are many reasons for pressure fluctuation, such as abnormal shaking of the pipeline or the operator using hemostatic forceps to clamp the extracorporeal circulation pipeline or quickly adjust the flow rate, which will cause pressure fluctuation.

The inventors have found that when the pipeline shakes and causes pressure fluctuations, the maximum change in pressure value does not exceed 4.0 kPa (30 mmHg). When the pipeline is clamped with hemostatic forceps or the flow rate is quickly adjusted, the pressure value usually changes above 8.0 kPa (60 mmHg). The maximum deviation values of pressure fluctuations in the above two situations are different. Therefore, in order to accurately distinguish the above two situations, the present invention further obtains the maximum deviation value of pressure fluctuations in the pressure sampling data.

In one embodiment, the above step S24 of "obtaining the maximum deviation value of the pressure fluctuation in the pressure sampling data" specifically includes: obtaining the maximum value of the difference between all abnormal pressure values in the pressure sampling data and the corresponding regression pressure values as the maximum deviation value of the pressure fluctuation in the pressure sampling data.

In a specific implementation, the abnormal pressure value in the pressure sampling data is determined. Specifically, each sampling time corresponds to a pressure value obtained by actual sampling and a regression pressure value calculated according to the linear regression equation. If the difference between the pressure value and the regression pressure value (the difference here refers to the absolute value of the difference between the pressure value and the regression pressure value, that is, the degree of deviation between the pressure value and the linear regression equation) is greater than the preset pressure value threshold, the pressure value is determined to be an abnormal pressure value. In the present invention, the maximum value of the difference between all the abnormal pressure values in the pressure sampling data and the corresponding regression pressure value is used as the maximum deviation value of the pressure fluctuation in the pressure sampling data.

Step S25 (not shown): determining whether the maximum deviation value of the pressure fluctuation is greater than a preset deviation threshold.

In a specific implementation, the deviation threshold is set by those skilled in the art, and is not specifically limited in the present invention. For example, it can be set to 8.0 kPa (60 mmHg).

Step S26 (not shown): If the maximum deviation value of the pressure fluctuation is greater than a preset deviation threshold, it is determined that there is no abnormal pressure fluctuation in the extracorporeal circulation circuit within the target time range.

In a specific implementation, if the maximum deviation value of the pressure fluctuation is greater than the preset deviation threshold, it means that the pressure fluctuation is caused by the operator using hemostatic forceps to clamp the extracorporeal circulation pipeline or quickly adjust the flow rate. Therefore, it is determined that there is no abnormal pressure fluctuation in the extracorporeal circulation pipeline within the target time range.

Step S27 (not shown): If the maximum deviation value of the pressure fluctuation is not greater than a preset deviation threshold, it is determined that abnormal pressure fluctuation exists in the extracorporeal circulation circuit within the target time range.

In a specific implementation, if the maximum deviation value of the pressure fluctuation is not greater than a preset deviation threshold, it indicates that the pressure fluctuation is caused by pipeline shaking, and therefore it is determined that abnormal pressure fluctuation exists in the extracorporeal circulation pipeline within the target time range.

Step S3: if there is abnormal pressure fluctuation in the extracorporeal circulation pipeline within the target time range, obtain the abnormal fluctuation time range corresponding to the abnormal pressure fluctuation in the extracorporeal circulation pipeline.

In a specific implementation, if there is abnormal pressure fluctuation in the extracorporeal circulation pipeline within the target time range, the abnormal fluctuation time range corresponding to the abnormal pressure fluctuation in the extracorporeal circulation pipeline is obtained. Usually, the abnormal fluctuation time range corresponds to a large pressure fluctuation in the extracorporeal circulation pipeline, so the abnormal fluctuation time range can be determined by the difference between the pressure value and the regression pressure value.

For example, in one embodiment, the fitting result also includes a linear regression equation, and the above step S3 of "obtaining the abnormal fluctuation time range corresponding to the abnormal pressure fluctuation in the extracorporeal circulation pipeline" specifically includes the following steps:

Step S31 (not shown): calculating the regression pressure value corresponding to the sampling time based on the linear regression equation.

In a specific implementation, each sampling time corresponds to a pressure value actually sampled and a regression pressure value calculated according to a linear regression equation.

Step S32 (not shown): If the difference between the pressure value corresponding to the sampling time and the regression pressure value is greater than a preset pressure value threshold, determine that the pressure value corresponding to the sampling time is an abnormal pressure value.

In a specific implementation, the difference between the pressure value corresponding to the sampling time and the regression pressure value is calculated (the difference here refers to the absolute value of the difference between the pressure value and the regression pressure value, that is, the degree of deviation between the pressure value and the linear regression equation is characterized). If the difference between the pressure value corresponding to the sampling time and the regression pressure value is greater than a preset pressure value threshold, the pressure value is determined to be an abnormal pressure value. The pressure value threshold is set by those skilled in the art and is not specifically limited in the present invention.

Step S33 (not shown): taking the sampling time corresponding to the earliest abnormal pressure value within the target time range as the starting point of the abnormal fluctuation time range, and taking the sampling time corresponding to the latest abnormal pressure value within the target time range as the end point of the abnormal fluctuation time range.

In a specific implementation, the sampling time corresponding to the earliest abnormal pressure value appearing within the target time range is the starting point of the abnormal pressure fluctuation, and thus can be used as the starting point of the abnormal fluctuation time range; the sampling time corresponding to the latest abnormal pressure value appearing within the target time range is the end point of the abnormal pressure fluctuation, and thus can be used as the end point of the abnormal fluctuation time range.

Step S4: determining whether the first moment is within the abnormal fluctuation time range.

In a specific implementation, it is determined whether the first moment is within the abnormal fluctuation time range, that is, it is determined whether there is abnormal pressure fluctuation caused by shaking in the extracorporeal circulation pipeline when the bubble signal is received.

Step S5: If the first moment is within the abnormal fluctuation time range, ignore the bubble signal.

In a specific implementation, if the first moment is within the abnormal fluctuation time range, it means that when the bubble signal is received, there is abnormal pressure fluctuation in the extracorporeal circulation pipeline due to shaking, which means that the bubble signal is very likely to be a misjudgment. Therefore, the bubble signal is ignored and there is no need to issue a bubble alarm message.

Step S6: If the first moment is not within the abnormal fluctuation time range, or if there is no abnormal pressure fluctuation in the extracorporeal circulation circuit within the target time range, a bubble alarm message is issued.

In a specific implementation, if the first moment is not within the abnormal fluctuation time range, or there is no abnormal pressure fluctuation in the extracorporeal circulation circuit within the target time range, it means that the bubble signal is not a misjudgment but is true and reliable. Therefore, a bubble alarm message is issued to remind the operator of the existence of bubbles.

The technical solution of the present invention, when receiving the bubble signal sent by the bubble sensor, determines based on the pressure information of the extracorporeal circulation pipeline whether there is abnormal pressure fluctuation, that is, pressure fluctuation caused by shaking, in the extracorporeal circulation pipeline at the first moment when the bubble signal is detected. If so, the bubble signal is ignored and no bubble alarm information is issued, thereby effectively avoiding false alarms of bubble warnings and greatly improving the accuracy of bubble detection.

Referring to FIG. 2, in a solution of an embodiment, the pressure values of multiple positions in the extracorporeal circulation circuit can be collected simultaneously, and each position can be judged separately, and its confidence can be judged according to the distance between the pressure collection position and the bubble sensor. For example, the pressure values of the first pressure sensor 3, the second pressure sensor 5 and the third pressure sensor 7 can be monitored at the same time. When a bubble signal is detected, the pressure values of the first pressure sensor 3, the second pressure sensor 5 and the third pressure sensor 7 are judged separately. If it is judged that there are pressure value fluctuations at all three positions, it means that the fluctuations are large, and it is judged as a false alarm caused by the shaking of the bubble sensor 8; if it is judged that only the third pressure sensor 7 has pressure value fluctuations, since the third pressure sensor 7 is closest to the bubble sensor 8, it can still be judged as a false alarm caused by the shaking of the sensor; if it is judged that only the second pressure sensor 5 has pressure value fluctuations or the first pressure sensor 3 has pressure value fluctuations, since the second pressure sensor 5 and the first pressure sensor 3 are far away from the bubble sensor, and the third pressure sensor 7, which is closer, does not have pressure fluctuations, it can be judged that it is not a false alarm caused by the shaking of the sensor, and the bubble alarm should be triggered.

In one embodiment, in order to further improve the accuracy of bubble detection, a flow sensor is further provided on the extracorporeal circulation pipeline of the extracorporeal circulation device, and the flow sensor is used to further assist in determining whether there is a misjudgment of the bubble sensor. Specifically, before the above step S5 of "ignoring the bubble signal", the method further includes the following steps:

Step S61 (not shown): acquiring flow sampling data of the flow sensor within the abnormal fluctuation time range.

In a specific implementation, the flow sensor collects the flow value of the extracorporeal circulation circuit once every preset pressure sampling period. Therefore, the flow sampling data of the flow sensor within the abnormal fluctuation time range can be obtained. The flow sampling data includes multiple flow values corresponding to different sampling times.

Step S62 (not shown): Based on the flow sampling data, determine whether there is flow fluctuation in the extracorporeal circulation circuit within the abnormal fluctuation time range.

In a specific implementation, under normal circumstances, the flow rate on the extracorporeal circulation pipeline also presents a linear change, so a linear fitting is performed through the flow sampling data, that is, the sampling time in the flow sampling data is used as the independent variable, and the flow value is used as the dependent variable, and a linear fitting is performed to obtain a correlation coefficient. According to the size of the correlation coefficient, it can be judged whether the flow rate on the extracorporeal circulation pipeline presents a linear change over time. Specifically, if the difference between the absolute value of the correlation coefficient and 1 (the difference here refers to the absolute value of the difference between the absolute value of the correlation coefficient and 1) is less than a preset difference threshold, it is determined that the flow rate on the extracorporeal circulation pipeline presents a linear change over time, otherwise, it is determined that the flow rate on the extracorporeal circulation pipeline presents a nonlinear change over time.

Furthermore, if the flow rate in the extracorporeal circulation pipeline presents a nonlinear change over time, it is determined that there is flow rate fluctuation in the extracorporeal circulation pipeline within the abnormal fluctuation time range.

If the flow rate on the extracorporeal circulation pipeline changes linearly with time, it is determined that there is no flow rate fluctuation in the extracorporeal circulation pipeline within the abnormal fluctuation time range.

Step S63 (not shown): If there is flow fluctuation in the extracorporeal circulation pipeline within the abnormal fluctuation time range, a bubble alarm message is issued.

In a specific implementation, if there is flow fluctuation in the extracorporeal circulation pipeline within the abnormal fluctuation time range, it means that the pressure fluctuation is caused by the flow fluctuation. Then the bubble signal detected at this time is generated by real bubbles in the pipeline, so a bubble alarm message is issued.

Furthermore, if there is no flow fluctuation in the extracorporeal circulation pipeline within the abnormal fluctuation time range, it means that the pressure fluctuation is caused by shaking. Then the bubble signal detected at this time is caused by pipeline shaking, and therefore the step of ignoring the bubble signal is executed.

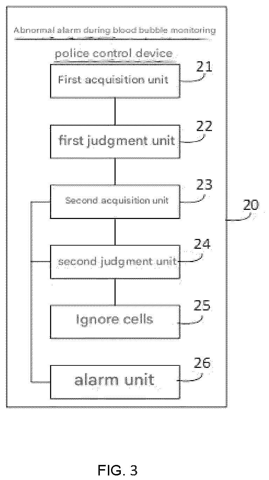

FIG. 3 shows a schematic block diagram of a control device 20 for abnormal alarm in blood bubble monitoring provided by an embodiment of the present invention. Corresponding to the above control method for abnormal alarm in blood bubble monitoring, the present invention also provides a control device 20 for abnormal alarm in blood bubble monitoring. The control device 20 for abnormal alarm in blood bubble monitoring includes a unit for executing the above control method for abnormal alarm in blood bubble monitoring, and the control device 20 for abnormal alarm in blood bubble monitoring can be configured in a desktop computer, a tablet computer, a laptop computer, and other terminals. Specifically, the control device 20 for abnormal alarm in blood bubble monitoring includes:

A first acquisition unit 21 is used to acquire a first moment when the bubble signal is generated upon receiving the bubble signal sent by the bubble sensor, and acquire pressure sampling data of the extracorporeal circulation pipeline within a preset target time range based on the pressure acquisition device, wherein the first moment is within the target time range;

A first judgment unit 22 is used to judge whether there is abnormal pressure fluctuation in the extracorporeal circulation circuit within the target time range based on the pressure sampling data;

A second acquisition unit 23 is used for acquiring an abnormal fluctuation time range corresponding to the abnormal pressure fluctuation in the extracorporeal circulation pipeline if the abnormal pressure fluctuation exists in the extracorporeal circulation pipeline within the target time range;

A second judgment unit 24 is used to judge whether the first moment is within the abnormal fluctuation time range;

An ignoring unit 25, configured to ignore the bubble signal if the first moment is within the abnormal fluctuation time range;

An alarm unit 26 is used to issue a bubble alarm message if the first moment is not within the abnormal fluctuation time range, or if there is no abnormal pressure fluctuation in the extracorporeal circulation circuit within the target time range.

In one embodiment, the pressure sampling data includes pressure values at multiple different sampling times, and judging whether there is abnormal pressure fluctuation in the extracorporeal circulation circuit within the target time range based on the pressure sampling data includes:

Performing linear fitting based on the sampling time and pressure value in the pressure sampling data to obtain a fitting result;

Determining, according to the fitting result, whether the pressure value in the pressure sampling data changes linearly with the sampling time;

If the pressure value in the pressure sampling data changes linearly with the sampling time, it is determined that there is no abnormal pressure fluctuation in the extracorporeal circulation circuit within the target time range.

In one embodiment, judging whether there is abnormal pressure fluctuation in the extracorporeal circulation circuit within the target time range based on the pressure sampling data further includes:

If the pressure value in the pressure sampling data presents a nonlinear change with the sampling time, obtaining a maximum deviation value of the pressure fluctuation in the pressure sampling data;

Determining whether the maximum deviation value of the pressure fluctuation is greater than a preset deviation threshold;

If the maximum deviation value of the pressure fluctuation is greater than a preset deviation threshold, it is determined that there is no abnormal pressure fluctuation in the extracorporeal circulation circuit within the target time range;

If the maximum deviation value of the pressure fluctuation is not greater than a preset deviation threshold, it is determined that abnormal pressure fluctuation exists in the extracorporeal circulation circuit within the target time range.

In one embodiment, the fitting result includes a correlation coefficient, and judging whether the pressure value in the pressure sampling data presents a linear change with the sampling time according to the fitting result includes:

Determine whether the difference between the absolute value of the correlation coefficient and 1 is less than a preset difference threshold;

If the difference between the absolute value of the correlation coefficient and 1 is less than a preset difference threshold, it is determined that the pressure value in the pressure sampling data changes linearly with the sampling time;

If the difference between the absolute value of the correlation coefficient and 1 is not less than a preset difference threshold, it is determined that the pressure value in the pressure sampling data presents a nonlinear change with the sampling time.

In one embodiment, the fitting result further includes a linear regression equation, and the abnormal fluctuation time range corresponding to the abnormal pressure fluctuation in the extracorporeal circulation circuit is obtained, including:

Calculate the regression pressure value corresponding to the sampling time based on the linear regression equation;

If the difference between the pressure value corresponding to the sampling time and the regression pressure value is greater than a preset pressure value threshold, it is determined that the pressure value corresponding to the sampling time is an abnormal pressure value;

The sampling time corresponding to the earliest abnormal pressure value within the target time range is taken as the starting point of the abnormal fluctuation time range, and the sampling time corresponding to the latest abnormal pressure value within the target time range is taken as the end point of the abnormal fluctuation time range.

In one embodiment, the obtaining of the maximum deviation value of the pressure fluctuation in the pressure sampling data includes:

The maximum value of the difference between all abnormal pressure values and the corresponding regression pressure values is obtained as the maximum deviation value of the pressure fluctuation in the pressure sampling data.

In one embodiment, the pressure acquisition device includes a plurality of pressure sensors, and the pressure sampling data of the extracorporeal circulation circuit within a preset target time range is obtained based on the pressure acquisition device, including:

From the plurality of pressure sensors, obtaining a pressure sensor that is closest to the bubble sensor as a target pressure sensor;

The pressure data of the target pressure sensor within a preset target time range is acquired as the pressure sampling data.

In one embodiment, a flow sensor is further provided on the extracorporeal circulation pipeline of the extracorporeal circulation device, and the control device 20 for abnormal alarm during blood bubble monitoring further includes:

A third acquisition unit, used to acquire flow sampling data of the flow sensor within the abnormal fluctuation time range;

A third judgment unit is used to judge whether there is flow fluctuation in the extracorporeal circulation pipeline within the abnormal fluctuation time range based on the flow sampling data;

The alarm unit is further configured to issue a bubble alarm message if there is flow fluctuation in the extracorporeal circulation pipeline within the abnormal fluctuation time range;

The ignoring unit is further configured to ignore the bubble signal if there is no flow fluctuation in the extracorporeal circulation pipeline within the abnormal fluctuation time range.

A further technical solution is that the pressure sampling period within the target time range is 100-1000 milliseconds, and the length of the target time range is 5-90 seconds.

It should be noted that technicians in the relevant field can clearly understand that the specific implementation process of the control device 20 and each unit of the abnormal alarm in the above-mentioned blood bubble monitoring can refer to the corresponding description in the aforementioned method embodiment, and for the convenience and brevity of description, it will not be repeated here.

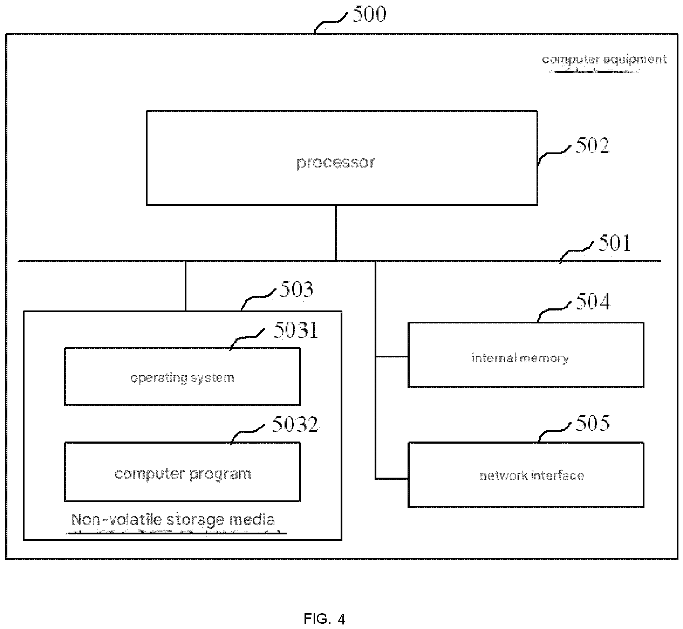

The control device 20 for abnormal alarm in the above-mentioned blood bubble monitoring can be implemented in the form of a computer program, and the computer program can be run on the computer device shown in FIG. 4.

FIG. 4 is a schematic block diagram of a computer device provided in an embodiment of the present application. The computer device 500 can be a terminal or a server, wherein the terminal can be an electronic device with communication functions such as a smart phone, a tablet computer, a laptop computer, a desktop computer, a personal digital assistant, and a wearable device. The server can be an independent server or a server cluster composed of multiple servers.

The computer device 500 includes a processor 502, a memory, and a network interface 505 connected via a system bus 501, wherein the memory may include a non-volatile storage medium 503 and an internal memory 504.

The non-volatile storage medium 503 can store an operating system 5031 and a computer program 5032. When the computer program 5032 is executed, the processor 502 can execute a control method for abnormal alarm in blood bubble monitoring.

The processor 502 is used to provide computing and control capabilities to support the operation of the entire computer device 500.

The internal memory 504 provides an environment for the operation of the computer program 5032 in the non-volatile storage medium 503. When the computer program 5032 is executed by the processor 502, the processor 502 can execute a control method for abnormal alarm in blood bubble monitoring.

The network interface 505 is used to communicate with other devices over the network. Those skilled in the art will appreciate that the above structure is only a block diagram of a portion of the structure related to the present application solution, and does not constitute a limitation on the computer device 500 to which the present application solution is applied. The specific computer device 500 may include more or fewer components than those shown in the figure, or combine certain components, or have a different arrangement of components.

A computer program 5032 stored in a memory to implement a control method for abnormal alarm in blood bubble monitoring provided by any of the above method embodiments.

It should be understood that in the embodiment of the present application, the processor 502 may be a central processing unit (CPU), and the processor 502 may also be other general-purpose processors, digital signal processors (DSP), application-specific integrated circuits (ASIC), field-programmable gate arrays (FPGA) or other programmable logic devices, discrete gate or transistor logic devices, discrete hardware components, etc. Among them, the general-purpose processor may be a microprocessor or the processor may also be any conventional processor, etc.

It is understood by those skilled in the art that all or part of the processes in the method for implementing the above embodiment can be completed by instructing the relevant hardware through a computer program. The computer program can be stored in a storage medium, which is a computer-readable storage medium. The computer program is executed by at least one processor in the computer system to implement the process steps of the embodiment of the above method.

Therefore, the present invention also provides a storage medium. The storage medium may be a computer-readable storage medium. The storage medium stores a computer program. When the computer program is executed by a processor, the processor executes a control method for abnormal alarm in blood bubble monitoring provided by any of the above method embodiments.

The storage medium is a physical, non-transient storage medium, such as a USB flash drive, a mobile hard disk, a read-only memory (ROM), a magnetic disk, or an optical disk, etc., which can store program codes. The computer-readable storage medium can be non-volatile or volatile.

Those of ordinary skill in the art will appreciate that the units and algorithm steps of each example described in conjunction with the embodiments disclosed herein can be implemented in electronic hardware, computer software, or a combination of the two. In order to clearly illustrate the interchangeability of hardware and software, the composition and steps of each example have been generally described in terms of function in the above description. Whether these functions are performed in hardware or software depends on the specific application and design constraints of the technical solution. Professional and technical personnel can use different methods to implement the described functions for each specific application, but such implementation should not be considered to be beyond the scope of the present invention.

In the several embodiments provided by the present invention, it should be understood that the disclosed devices and methods can be implemented in other ways. For example, the device embodiments described above are only schematic. For example, the division of each unit is only a logical function division, and there may be other division methods in actual implementation. For example, multiple units or components can be combined or integrated into another system, or some features can be ignored or not executed.

The steps in the method of the embodiment of the present invention can be adjusted in order, combined and deleted according to actual needs. The units in the device of the embodiment of the present invention can be combined, divided and deleted according to actual needs. In addition, the functional units in the various embodiments of the present invention can be integrated into one processing unit, or each unit can exist physically separately, or two or more units can be integrated into one unit.

If the integrated unit is implemented in the form of a software functional unit and sold or used as an independent product, it can be stored in a storage medium. Based on this understanding, the technical solution of the present invention is essentially or the part that contributes to the prior art, or all or part of the technical solution can be embodied in the form of a software product. The computer software product is stored in a storage medium and includes several instructions for a computer device (which can be a personal computer, terminal, or network device, etc.) to perform all or part of the steps of the method described in each embodiment of the present invention.

In the above embodiments, the description of each embodiment has its own emphasis. For parts that are not described in detail in a certain embodiment, reference can be made to the relevant descriptions of other embodiments.

Obviously, those skilled in the art can make various changes and modifications to the present invention without departing from the spirit and scope of the present invention. Thus, if these modifications and variations of the present invention fall within the scope of the claims of the present invention and their equivalents, the present invention is also intended to include these modifications and variations.

The above is only a specific embodiment of the present invention, but the protection scope of the present invention is not limited thereto. Any technician familiar with the technical field can easily think of various equivalent modifications or replacements within the technical scope disclosed by the present invention, and these modifications or replacements should be included in the protection scope of the present invention. Therefore, the protection scope of the present invention shall be based on the protection scope of the claims.

## Claims

1. A control method for abnormal alarm in blood bubble monitoring, **characterized in that** a bubble sensor and a pressure acquisition device are provided on the extracorporeal circulation pipeline of an extracorporeal circulation device, and the method comprises:
obtaining the first moment when the bubble signal is generated when receiving the bubble signal sent by the bubble sensor, and obtaining the pressure sampling data of the extracorporeal circulation pipeline within a preset target time range based on the pressure acquisition device, wherein the first moment is within the target time range;
determining whether there is abnormal pressure fluctuation in the extracorporeal circulation circuit within the target time range based on the pressure sampling data;
obtaining an abnormal fluctuation time range corresponding to the abnormal pressure fluctuation in the extracorporeal circulation pipeline in response to an abnormal pressure fluctuation being in the extracorporeal circulation pipeline within the target time range;
determining whether the first moment is within the abnormal fluctuation time range;
ignoring the bubble signal in response to the first moment being within the abnormal fluctuation time range;
Issuing bubble alarm message in response to the first moment not being within the abnormal fluctuation time range, or in response to there being no abnormal pressure fluctuation in the extracorporeal circulation circuit within the target time range.

2. The control method for abnormal alarm in blood bubble monitoring according to claim 1, **characterized in that** the pressure sampling data includes pressure values at multiple different sampling times, and judging whether there is abnormal pressure fluctuation in the extracorporeal circulation circuit within the target time range based on the pressure sampling data comprises:
performing linear fitting based on the sampling time and pressure value in the pressure sampling data to obtain a fitting result;
determining, according to the fitting result, whether the pressure value in the pressure sampling data changes linearly with the sampling time;
determining that there is no abnormal pressure fluctuation in the extracorporeal circulation circuit within the target time range in response to determining that the pressure value in the pressure sampling data changing linearly with the sampling time.

3. The control method for abnormal alarm in blood bubble monitoring according to claim 2, **characterized in that** the step of judging whether there is abnormal pressure fluctuation in the extracorporeal circulation circuit within the target time range based on the pressure sampling data further comprises:
obtaining a maximum deviation value of the pressure fluctuation in the pressure sampling data in response to the pressure value in the pressure sampling data presenting a nonlinear change with the sampling time;
determining whether the maximum deviation value of the pressure fluctuation is greater than a preset deviation threshold;
determining that there is no abnormal pressure fluctuation in the extracorporeal circulation circuit within the target time range based on the maximum deviation value of the pressure fluctuation being greater than a preset deviation threshold;
determining that abnormal pressure fluctuation exists in the extracorporeal circulation circuit within the target time range based on the maximum deviation value of the pressure fluctuation not being greater than a preset deviation threshold.

4. The control method for abnormal alarm in blood bubble monitoring according to claim 2, **characterized in that** the fitting result includes a correlation coefficient, and judging whether the pressure value in the pressure sampling data presents a linear change with the sampling time according to the fitting result comprises:
determining whether the difference between the absolute value of the correlation coefficient and 1 is less than a preset difference threshold;
determining that the pressure value in the pressure sampling data changes linearly with the sampling time in response to the difference between the absolute value of the correlation coefficient and 1 being less than a preset difference threshold;
determining that the pressure value in the pressure sampling data presents a nonlinear change with the sampling time in response to the difference between the absolute value of the correlation coefficient and 1 being not less than a preset difference threshold.

5. The control method for abnormal alarm in blood bubble monitoring according to claim 3, **characterized in that** the fitting result also includes a linear regression equation, and the abnormal fluctuation time range corresponding to the abnormal pressure fluctuation in the extracorporeal circulation pipeline is obtained, including:
calculating the regression pressure value corresponding to the sampling time based on the linear regression equation;
determining that the pressure value corresponding to the sampling time is an abnormal pressure value in response to the difference between the pressure value corresponding to the sampling time and the regression pressure value being greater than a preset pressure value threshold;
setting the sampling time corresponding to the earliest abnormal pressure value within the target time range as the starting point of the abnormal fluctuation time range, and setting the sampling time corresponding to the latest abnormal pressure value within the target time range as the end point of the abnormal fluctuation time range.

6. The control method for abnormal alarm in blood bubble monitoring according to claim 5, **characterized in that** the step of obtaining the maximum deviation value of pressure fluctuation in the pressure sampling data comprises:
setting the maximum value of the difference between all abnormal pressure values and the corresponding regression pressure values as the maximum deviation value of the pressure fluctuation in the pressure sampling data.

7. The control method for abnormal alarm in blood bubble monitoring according to claim 1, **characterized in that** the pressure acquisition device comprises a plurality of pressure sensors, and the pressure sampling data of the extracorporeal circulation circuit within a preset target time range is obtained based on the pressure acquisition device, comprising:
setting a pressure sensor, from the plurality of pressure sensors, that is closest to the bubble sensor as a target pressure sensor;
setting the pressure data of the target pressure sensor within a preset target time range as the pressure sampling data.

8. The control method for abnormal alarm in blood bubble monitoring according to claim 1, **characterized in that** a flow sensor is also provided on the extracorporeal circulation pipeline of the extracorporeal circulation device, and before ignoring the bubble signal, the method further comprises:
acquiring flow sampling data of the flow sensor within the abnormal fluctuation time range;
determining whether there is flow fluctuation in the extracorporeal circulation pipeline within the abnormal fluctuation time range based on the flow sampling data;
issuing a bubble alarm message based on a flow fluctuation being in the extracorporeal circulation pipeline within the abnormal fluctuation time range;
ignoring the bubble alarm message based on there being no flow fluctuation in the extracorporeal circulation circuit within the abnormal fluctuation time range.

9. The control method for abnormal alarm in blood bubble monitoring according to claim 1, **characterized in that** the pressure sampling period within the target time range is 100 to 1000 milliseconds, and the length of the target time range is 5 to 90 seconds.

10. A control device for abnormal alarm in blood bubble monitoring, **characterized in that** it comprises a unit for executing the method according to any one of claims 1 to 9.

11. A computer device, **characterized in that** the computer device comprises:
a memory that stores a computer program; and
a processor connected to the memory that implements the method according to claim 1 when executing the computer program.

12. A computer-readable storage medium, **characterized in that** the storage medium stores a computer program, and when the computer program is executed by a processor to implement the method according to claim 1.
